# EUROPÄISCHE PATENTSCHRIFT

(11) **EP 2 419 092 B1**
(45) Veröffentlichungstag und Bekanntmachung des Hinweises auf die Patenterteilung: **04.05.2016**
(21) Anmeldenummer: 10714260.6
(22) Anmeldetag: 15.04.2010
(51) Int. Cl.: A61K 31/00, A61K 31/4439, A61K 47/06, A61K 47/14, A61K 47/22, A61K 47/44, A61P 17/06, A61P 17/00, A61P 37/00

(54) **PHARMAZEUTISCHE ZUSAMMENSETZUNG ZUR BEHANDLUNG VON DERMATOLOGISCHEN AUTOIMMUNERKRANKUNGEN**
PHARMACEUTICAL COMPOSITION FOR TREATING DERMATOLOGICAL AUTOIMMUNE DISEASES
PRÉPARATION PHARMACEUTIQUE POUR LE TRAITEMENT DE MALADIES DERMATOLOGIQUES AUTO-IMMUNES

(30) Priorität: 15.04.2009 DE 102009018133
(43) Veröffentlichungstag der Anmeldung: 22.02.2012
(73) Patentinhaber: Agon Pharma GmbH, 73240 Wendlingen (DE)
(72) Erfinder: STEPHAN, Günter, 70469 Stuttgart (DE); FARR, Charlie, A-1180 Wien (AT)
(74) Vertreter: Hoeger, Stellrecht & Partner Patentanwälte mbB
(86) Internationale Anmeldenummer: PCT/EP2010/054979
(87) Internationale Veröffentlichungsnummer: WO 2010/119102

(56) Entgegenhaltungen:
- WO-A1-95/18612
- WO-A1-96/31213
- WO-A1-2004/041280
- WO-A2-2005/097083

## Beschreibung

Die vorliegende Erfindung betrifft therapeutische Zusammensetzungen zur topischen Behandlung von dermatologischen Autoimmunerkrankungen, insbesondere zur Behandlung von atopischem Ekzem.

Das atopische Ekzem ist eine Erkrankung der Haut aus dem immunologischen Formenkreis. Es wird synonym auch als Neurodermitis, atopische Dermatitis, endogenes Ekzem, chronisch konstitutionelles Ekzem, Asthmaekzem oder Prurigo Besnier bezeichnet. Von dieser Krankheit sind z.B. in Deutschland etwa 1 bis 3% der Erwachsenen betroffen, wobei seit der Mitte des 20. Jahrhunderts bis heute von einem 4- bis 6-mal häufigeren Auftreten des atopischen Ekzems ausgegangen wird. Besonders stark betroffen sind Kinder mit etwa 10 bis 20%; bei etwa 60% der Betroffenen tritt die Erkrankung im ersten Lebensjahr auf und verschwindet bei ca. 30 bis 50% dieser Patienten wieder im Verlauf der Kindheit.

Die Hauptsymptome des atopischen Ekzems sind rote, schuppende, manchmal auch nässende Ekzeme auf der Haut sowie ein starker, auch nachts anhaltender Juckreiz. Beim Säugling ist vor allem das Gesicht betroffen, später dehnt sich das atopische Ekzem typischerweise auf die großen Beugen, Hände, Füße und den Hals aus. Die beim Erwachsenen im Vordergrund stehenden Symptome sind trockene Haut, Lichenifikation und aufgekratzte Knoten.

Als primäre Ursache des atopischen Ekzems gilt heute eine Veranlagung als gesichert, d.h. eine genetische Störung sowohl der epidermalen als auch der immunologischen Barrierefunktion der Haut. Diese genetische Prädisposition führt in Wechselwirkung mit Umweltfaktoren sowie psychischen Einflüssen zu den Krankheitssymptomen.

Ebenso wie die Ursachen sind auch die angewandten Behandlungsformen sehr unterschiedlich, und nicht alle Behandlungsansätze wirken bei allen Betroffenen gleich. Mittelpunkt der symptomatischen Therapie ist die Basispflege der Haut in Form einer topischen Behandlung mit Salben, Cremes oder Lotionen, wodurch die Barrierefunktion der Haut stabilisiert und deren Empfindlichkeit gegenüber Irritationen und dem Eindringen von Allergenen abgeschwächt werden soll. So helfen insbesondere harnstoffhaltige Präparate, um die charakteristische Trockenheit der Haut zu reduzieren. Weitere häufig verwendete Wirkstoffe sind z.B. Nachtkerzenöl, Johanniskrautextrakt, Zink und Dexpanthenol.

Zur topischen Behandlung von stärkeren entzündlichen Symptomen werden Immunsuppressiva bzw. entzündungshemmende Wirkstoffe eingesetzt. Am häufigsten werden dabei Glucocorticoide verwendet, durch die schwere Schübe gelindert oder bei rechtszeitiger Anwendung vermieden werden können. Abhängig von der Art und Schwere der Symptome stehen sowohl stark wirksame Glucocorticoide (Klasse 3) als auch schwache oder mittelstarke Präparate (Klassen 1 und 2) zur Verfügung. Insbesondere bei großflächiger Anwendung von Glucocorticoiden können jedoch Nebenwirkungen in Form von Hautverdünnung (Atrophie), Pigmentierungsstörungen, starker Behaarung (Hypertrichose), Dehnungsstreifen sowie einer partiellen Unterdrückung des örtlichen Immunsystems auftreten. Daher sollte eine Behandlung mit Glucocorticoiden nur kurzzeitig erfolgen.

Als Alternative zu Glucocorticoiden sind seit wenigen Jahren die lokal anwendbaren Immunsuppressiva Tacrolimus und Pimecrolimus verfügbar. Beide Substanzen gehören zur Gruppe der Makrolide und wirken als Calcineurininhibitoren. Im Gegensatz zu Glucocorticoiden haben sie keine atrophisierenden Effekte und werden vor allem dort eingesetzt, wo die Haut sehr dünn ist und dadurch eine erhöhte Penetrationsgefahr der Wirkstoffe gegeben ist (Gesicht, Hals und Genitalbereich). Bisher liegen keine Langzeiterfahrungen dazu vor, ob diese Wirkstoffe zur Bildung von Tumoren beitragen, sodass die USamerikanische Arzneimittelbehörde FDA im Jahr 2005 eine entsprechende Warnung veröffentlichte. Auch die europäische Arzneimittelagentur EMEA schränkt den Gebrauch auf Fälle ein, bei denen ein ausreichender Therapieerfolg mit Glucocorticoiden nicht zu erreichen ist oder die Nebenwirkungen eine Glucocorticoid-Therapie nicht zulassen.

Wirkstoffe zur inneren Anwendung sind insbesondere Antihistaminika, die den Juckreiz lindern können. Dies wird in erster Linie bei Kindern eingesetzt, damit sie leichter einschlafen können und sich so weniger kratzen müssen. Die innere Anwendung von Cortison und Cyclosporin A ist aufgrund der erheblichen Nebenwirkungen auf schwere bzw. schwerste Formen des atopischen Ekzems beschränkt.

WO 95/18612 offenbart die Behandlung von Psoriasis mit Omeprazol, wobei Omeprazol oral, rektal oder parenteral verabreicht wird, um so einen systemischen Effekt zu erlangen.

Es wurde nun überraschenderweise festgestellt, dass Protonenpumpenhemmer bei der Behandlung von atopischem Ekzem und anderen dermatologischen Autoimmunerkrankungen wirksam sind.

Gegenstand der vorliegenden Erfindung ist eine pharmazeutische Zusammensetzung, umfassend einen Protonenpumpenhemmer, zur Verwendung in der Behandlung von dermatologischen Autoimmunerkrankungen, wobei die pharmazeutische Zusammensetzung topisch angewendet wird. Offenbart ist somit auch die Verwendung eines Protonenpumpenhemmers zur Behandlung von dermatologischen Autoimmunerkrankungen. Protonenpumpenhemmer (Protonenpumpeninhibitoren, PPI) sind Wirkstoffe, die bisher nur zur Behandlung von Erkrankungen eingesetzt wurden, bei denen eine Reduzierung der Magensäuresekretion angezeigt ist. Hierzu zählen insbesondere das Magengeschwür (Ulcus ventriculi), das Zwölffingerdarmgeschwür (Ulcus duodeni), die Refluxkrankheit der Speiseröhre (Refluxösophagitis) und das Zollinger-Ellison-Syndrom. Protonenpumpenhemmer blockieren die für die Sekretion der Magensäure zuständige Protonen-Kalium-Pumpe (H⁺/K⁺-ATPase) in den Belegzellen der Magenschleimhaut.

Der Protonenpumpenhemmer umfasst vorzugsweise ein substituiertes Pyridylmethylsulfinylbenzimidazol oder dessen pharmazeutisch annehmbares Salz, insbesondere eine Verbindung mit der allgemeinen Formel I: 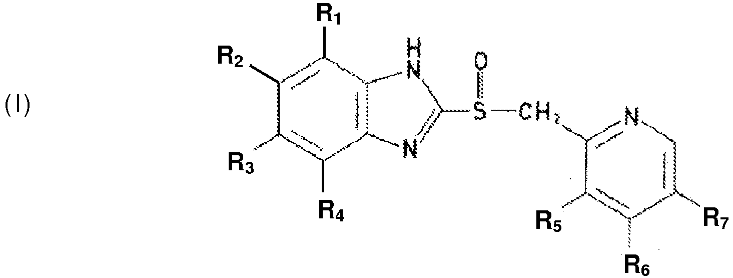 wobei R₁ bis R₄ gleich oder verschieden sind und jeweils ausgewählt sind aus Wasserstoff, Alkyl, Alkoxy, Halogen, Halogenalkoxy, Alkylcarbonyl, Alkoxycarbonyl, Oxazolyl und Trifluoralkyl, oder wobei benachbarte Gruppen R₁ bis R₄ Ringstrukturen, die weiter substituiert sein können, bilden; und wobei R₅, R₆ und R₇ gleich oder verschieden sind und jeweils ausgewählt sind aus Wasserstoff, Alkyl, gegebenenfalls durch Fluor substituiertem Alkoxy, Alkylthio, Alkoxyalkoxy, Dialkylamino, Piperidino, Morpholino, Halogen, Phenyl und Phenylalkoxy.

Bei den substituierten Pyridylmethylsulfinylbenzimidazolen handelt es sich im Rahmen ihres Einsatzes als Protonenpumpenhemmer um so genannte Prodrugs, die durch das saure Milieu im Magen in ein Sulfenamid umgewandelt werden, welches als eigentliche Wirksubstanz die Protonen-Kalium-Pumpe irreversibel hemmt. Sie werden daher als auch säureempfindliche Protonenpumpenhemmer bezeichnet. Daneben haben diese Verbindungen auch eine antibakterielle Wirkung gegen Helicobacter pylori.

Typische Vertreter der säureempfindlichen Protonenpumpenhemmer sind insbesondere Omeprazol mit der Form el II, 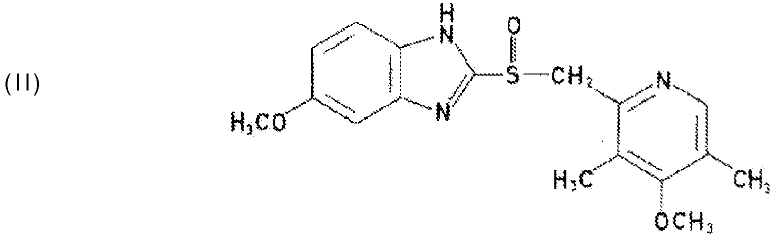 dessen reines (S)-Enantiomer auch als Esomeprazol bezeichnet wird,
Lansoprazol mit der Formel III, 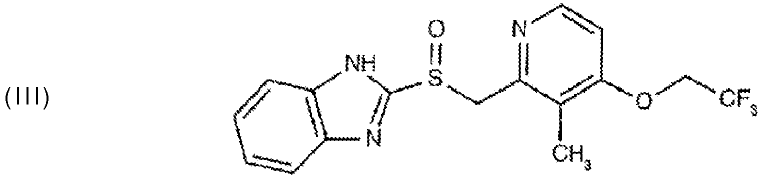 und Pantoprazol mit der Formel IV.

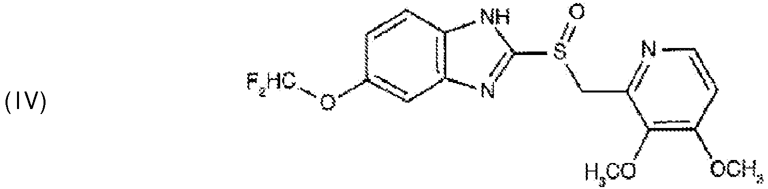

Die drei genannten Wirkstoffe weisen bei der Hemmung der Protonenpumpe einen identischen Wirkungsmechanismus auf, wobei die säureinduzierte Umwandlung von Pantoprazol bei deutlich niedrigeren pH-Werten erfolgt als im Fall von Omeprazol und Lansoprazol. Die Anwendung zur Reduzierung der Magensäuresekretion erfolgt systemisch, insbesondere peroral mittels magensaftresistenter Tabletten oder Kapseln, oder auch intravenös.

Die substituierten Pyridylmethylsulfinylbenzimidazole gehören zu den am häufigsten verordneten Medikamenten im gastrointestinalen Bereich, wobei Nebenwirkungen bei der oralen Einnahme nach bisherigem Kenntnisstand selten bis sehr selten sind.

Im Rahmen der vorliegenden Erfindung ist der Protonenpumpenhemmer daher vorzugsweise ausgewählt aus Omeprazol, Esomeprazol, Lansoprazol, Pantoprazol, deren pharmazeutisch annehmbaren Salzen, und Mischungen hiervon. Pharmazeutisch annehmbare Salze umfassen insbesondere die Natrium- und Magnesiumsalze der genannten Verbindungen.

Die dermatologischen Autoimmunerkrankungen, die mit Protonenpumpenhemmern im Rahmen der vorliegenden Erfindung wirksam behandelt werden können, umfassen insbesondere das oben beschriebene atopische Ekzem, sind jedoch nicht hierauf beschränkt. Eine weitere Indikation ist z.B. die Psoriasis (Schuppenflechte), bei der es sich ebenfalls um eine Entzündungsreaktion der Haut handelt, die unter anderem auf eine genetische Veranlagung zurückzuführen ist.

Die pharmazeutische Zusammensetzung gemäß der vorliegenden Erfindung kann nicht nur beim Menschen angewendet werden, sondern auch zur Behandlung von dermatologischen Autoimmunerkrankungen bei Tieren. Hier ist insbesondere das Sommerekzem bei Pferden zu nennen, für das ähnliche Ursachen vermutet werden wie für das atopische Ekzem beim Menschen.

Die erfindungsgemäße pharmazeutische Zusammensetzung ist zur topischen Anwendung bestimmt, d.h. zum äußerlichen Auftragen auf die betroffenen Bereiche der Haut. Generell ist bei einer topischen Anwendung von geringeren Nebenwirkungen auszugehen als bei einer systemischen Verabreichung, da eine hohe Wirkstoffkonzentration nur im entsprechenden Zielbereich vorliegt. Das Risiko von Nebenwirkungen bei den bekannten Protonenpumpenhemmern, welches bereits bei der systemischen Verabreichung gering ist, ist bei einer topischen Anwendung daher vernachlässigbar.

Bei der pharmazeutischen Zusammensetzung kann es sich grundsätzlich um ein- oder mehrphasige Systeme handeln, d.h. um homogene Systeme oder Emulsionen, die je nach Art der verwendeten Grundstoffe bzw. Trägersubstanzen als Salben, Cremes, Gele oder Lotionen bezeichnet werden. Gemäß einer bevorzugten Ausführungsform der Erfindung liegt die pharmazeutische Zusammensetzung in Form einer Salbe vor, d.h. als einphasiges System mit einer lipophilen Trägersubstanz.

Die pharmazeutische Zusammensetzung gemäß der vorliegenden Erfindung umfasst bevorzugt 0,1 bis 20 Gew.% des Protonenpumpenhemmers, weiter bevorzugt 0,3 bis 10 Gew.%, und am meisten bevorzugt 1 bis 5 Gew.%. In der Regel können bereits mit Wirkstoffkonzentrationen am unteren Ende dieses Bereiches, also z.B. mit einer Wirkstoffkonzentration von ca. 1 Gew.%, gute Ergebnisse erzielt werden.

Günstig ist es, wenn die pharmazeutische Zusammensetzung eine lipophile Trägersubstanz als Salbengrundstoff umfasst. Bevorzugt ist die lipophile Trägersubstanz ausgewählt aus synthetischen und mineralischen Wachsen, Fetten und Ölen und deren synthetischen Derivaten, sowie Mischungen hiervon. Beispiele für synthetische oder mineralische Trägersubstanzen sind Polyethylenglykole, aliphatische Kohlenwasserstoffe (z.B. verschiedene Paraffine und Vaselin) und Silikone, die jeweils in unterschiedlichen Molekulargewichtsbereichen verfügbar sind.

Alternativ oder zusätzlich kann die pharmazeutische Zusammensetzung auch eine lipophile Trägersubstanz umfassen, die ausgewählt ist aus tierischen und pflanzlichen Wachsen, Fetten und Ölen und deren hydrierten oder teilhydrierten Derivaten, sowie Mischungen hiervon, wie z.B. Bienenwachs, Wollwachs oder Pflanzenöle.

Die pharmazeutische Zusammensetzung ist bevorzugt im Wesentlichen wasserfrei, da die als Protonenpumpenhemmer einsetzbaren Pyridylmethylsulfinylbenzimidazole nicht nur säureempfindlich, sondern auch generell wasserempfindlich sind. Insbesondere ist die oben beschriebene, säurekatalysierte Umwandlung in die entsprechenden Sulfenamide im Rahmen der Anwendung gemäß der vorliegenden Erfindung unerwünscht. Durch die Verwendung von wasserfreien Systemen kann daher die Stabilität und Haltbarkeit der erfindungsgemäßen pharmazeutischen Zusammensetzung erhöht werden.

Andererseits hat sich gezeigt, dass die pharmazeutische Zusammensetzung gemäß der Erfindung auch als zweiphasiges System mit einem Wasseranteil von bis zu 40 Gew.% mit einer ausreichenden Stabilität formuliert werden kann. Die pharmazeutische Zusammensetzung ist gemäß dieser weiteren bevorzugten Ausführungsform der Erfindung eine w/o-Emulsion oder eine o/w-Emulsion. Die Zusammensetzung kann in diesem Fall auch einen oder mehrere Emulgatoren umfassen.

Durch den Zusatz von geeigneten Hilfsstoffen kann der Säureempfindlichkeit der Protonenpumpenhemmer zusätzlich Rechnung getragen werden. Um einen pH-Wert der verwendeten Trägersubstanz im alkalischen Bereich einzustellen (bevorzugt im Bereich von ca. 8,5 bis 9,5), umfasst die pharmazeutische Zusammensetzung günstigerweise eine alkalische Substanz (z.B. eine alkoholische Kaliumhydroxidlösung), vorzugsweise in einer Menge von 0,1 bis 5 Gew.%.

Auch ist es vorteilhaft, wenn die pharmazeutische Zusammensetzung eine oder mehrere Puffersubstanzen umfasst, bevorzugt in einer Menge von bis zu 5 Gew.%. Hierbei kann es sich bei einer wasserfreien Zusammensetzung insbesondere um lipophile Amine wie z.B. Stearylamin handeln, die in einer lipophilen Trägersubstanz löslich sind und dem Abpuffern von Protonen dienen, oder bei einem zweiphasigen System z.B. um Trometamol.

Zur Bindung von eventuell vorhandenem Restwasser in einer wasserfreien pharmazeutischen Zusammensetzung kann diese Siliciumdioxid umfassen, bevorzugt in einer Menge von 0,1 bis 3 Gew.%.

Um einer oxidativen Zerstörung des Protonenpumpenhemmers vorzubeugen und die Stabilität der pharmazeutischen Zusammensetzung zu erhöhen, kann diese zusätzlich ein oder mehrere lipophile Antioxidantien umfassen, bevorzugt in einer Menge von bis zu 5 Gew.%, insbesondere von 0,01 bis 2 Gew.%. Geeignete lipophile Antioxidantien sind z.B. Vitamin E, Butylhydroxytoluol (BHT), Butylhydroxyanisol (BHA) und Ascorbylpalmitat.

Um ein alkalisches Milieu in der pharmazeutischen Zusammensetzung sicherzustellen, ist es alternativ oder zusätzlich zu den oben beschriebenen Maßnahmen vorteilhaft, wenn eine Konditionierung mit einem alkalischen Medium durchgeführt wird.

Gegenstand der vorliegenden Erfindung ist daher auch ein Verfahren zur Herstellung einer pharmazeutischen Zusammensetzung, umfassend einen Protonenpumpenhemmer, zur topischen Anwendung, wobei der Protonenpumpenhemmer mit einer lipophilen Trägersubstanz gemischt und diese anschließend mit einem alkalischen Medium behandelt wird. Bei der pharmazeutischen Zusammensetzung handelt es sich in diesem Fall um eine Salbe mit der lipophilen Trägersubstanz als Salbengrundstoff, wie dies oben beschrieben wurde.

Das alkalische Medium, mit dem die lipophile Trägersubstanz behandelt wird, umfasst bevorzugt eine wässrige alkalische Lösung, also z.B. eine wässrige Natrium- oder Kaliumhydroxidlösung. Ferner kann das alkalische Medium einen oder mehrere Stabilisatoren und/oder Antioxidantien umfassen, wie z.B. Natriumdisulfid oder Natrium-EDTA.

Günstig ist es, wenn die lipophile Trägersubstanz mit dem alkalischen Medium ausgekocht wird. Anschließend wird das alkalische Medium vorzugsweise entfernt.

Der oder die oben beschriebenen Hilfsstoffe können der lipophilen Trägersubstanz vor oder nach der Behandlung mit dem alkalischen Medium zugesetzt werden.

Günstigerweise wird das Verfahren zur Herstellung der pharmazeutischen Zusammensetzung ganz oder teilweise unter Schutzgasatmosphäre, z.B. Stickstoff oder Argon, durchgeführt. Hierdurch kann eine Oxidation des Protonenpumpenhemmers vermieden oder zumindest verringert werden. Insbesondere das Abkühlen der lipophilen Trägersubstanz nach dem Auskochen mit dem alkalischen Medium sowie die weitere Verarbeitung erfolgen bevorzugt unter Schutzgasatmosphäre.

Auch das Abfüllen der pharmazeutischen Zusammensetzung wird vorzugsweise unter Schutzgasatmosphäre durchgeführt. Geeignete Behältnisse umfassen insbesondere Tuben aus Aluminium, Kunststoff oder einem Verbundmaterial, z.B. Aluminiumtuben mit Septum, Polyfoiltuben (Kunststoff/ Aluminium/Kunststoff), Multiplexverbundtuben oder gemäß dem Blow-Seal-Verfahren hergestellte Kunststoffbehältnisse.

Die Tuben oder sonstige Behältnisse für die pharmazeutische Zusammensetzung können eine Innenbeschichtung aufweisen, die Antioxidantien als zusätzlichen Produktschutz enthält.

Als alternative Darreichungsform der pharmazeutischen Zusammensetzung kann gemäß einer weiteren Ausführungsform der Erfindung auch vorgesehen sein, dass der Protonenpumpenhemmer bzw. die Zusammensetzung in eine Polymerfolie integriert wird, die auf die betroffene Hautstelle aufgebracht wird. Besonders vorteilhaft ist es, wenn dabei eine kontinuierliche Wirkstoffabgabe aus der Polymerfolie erfolgt.

Diese und weitere Vorteile der Erfindung werden anhand der nachfolgenden Beispiele näher erläutert.

### Beispiele

### Herstellung von pharmazeutischen Zusammensetzungen mit einem Protonenpumpenhemmer zur topischen Anwendung

### Rezepturen 1 bis 4: wasserfreies System

Es wurden verschiedene pharmazeutische Zusammensetzungen gemäß der vorliegenden Erfindung (Rezepturen 1 bis 4) in Form von Salben hergestellt. Als Protonenpumpenhemmer enthielten alle Zusammensetzung mikronisiertes Omeprazol, sowie verschiedene Mischungen lipophiler Trägersubstanzen als Salbengrundstoff (Vaseline, verschiedene Paraffine, Plastibase DAC, mittelkettige Triglyceride und Bienenwachs). Ferner enthielten die Zusammensetzungen öllösliches Vitamin E und Butylhydroxytoluol als lipophile Antioxidantien und gegebenenfalls Stearylamin als lipophile Puffersubstanz.

Die genaue Zusammensetzung der Rezepturen 1 bis 4 ist in der nachfolgenden Tabelle 1 angegeben, wobei sämtliche Angaben als Gew.% zu verstehen sind.

**Tabelle 1**

| | Rezeptur 1 | Rezeptur 2 | Rezeptur 3 | Rezeptur 4 |
|---|---|---|---|---|
| Om eprazol | 1,00 | 1,00 | 1,00 | 1,00 |
| Vaseline | 82,98 | 72,98 | 82,98 | - |
| Paraffin subliquidum | 5,00 | - | - | - |
| Paraffinöl | - | - | 10,00 | 9,00 |
| Paraffin hart | - | - | 5,00 | - |
| Plastibase DAC | - | - | - | 88,98 |
| Mittelkettige Triglyceride | 5,00 | 15,00 | - | - |
| Bienenwachs | 5,00 | 8,00 | - | - |
| Vitamin E | 1,00 | 1,00 | 1,00 | 1,00 |
| Butylhydroxytoluol | 0,02 | 0,02 | 0,02 | 0,02 |
| Stearylamin | - | 2,00 | - | - |

Als weitere Antioxidantien kommen z.B. Butylhydroxyanisol (0,01 bis 0,5 Gew.%) und Ascorbylpalmitat (0,01 bis 0,2 Gew.%) in Frage.

### Rezeptur 5: w/o-Emulsion

Eine weitere pharmazeutische Zusammensetzung gemäß der vorliegenden Erfindung (Rezeptur 5) wurde in Form eines zweiphasigen Systems (w/o-Emulsion) hergestellt. Die genaue Zusammensetzung ist in der nachfolgenden Tabelle 2 angegeben, wobei sämtliche Angaben als Gew.% zu verstehen sind.

**Tabelle 2**

| | Rezeptur 5 |
|---|---|
| Paraffin subliquidum | 5,0 |
| Wollwachs | 8,0 |
| Vaseline | 40,0 |
| Mittelkettige Triglyceride | 7,5 |
| Vitamin-E-Acetat | 5,0 |
| Om eprazol | 1,0 |
| Butylhydroxytoluol | 0,2 |
| Wasser | 33,0 |
| Trometamol | 0,3 |

Zur Herstellung wurden zunächst die lipophilen Trägersubstanzen (Paraffin subliquidum, Wollwachs, Vaseline und mittelkettige Triglyceride) bei 80 °C geschmolzen. Das Trometamol wurde in dem Wasser gelöst und in die lipophilen Trägersubstanzen eingearbeitet und homogenisiert. Nach dem Kaltrühren auf ca. 25 bis 30 °C wurden das in dem Vitamin-E-Acetat gelöste bzw. suspendierte Omeprazol und Butylhydroxytoluol eingearbeitet.

### Behandlung von dermatologischen Autoimmunerkrankungen

Die Wirkung des Protonenpumpenhemmers Omeprazol in Form einer 1 Gew.-%igen Salbe gemäß der obigen Rezeptur 5 wurde bei zehn Patienten im Alter von 10 bis 35 Jahren, die seit mehreren Jahren an atopischem Ekzem litten und akute Symptome aufwiesen, untersucht.

Die Behandlung erfolgte durch Auftragen der Salbe auf die betroffenen Bereiche der Haut, insbesondere im Bereich der Ellenbogen und Kniekehlen, je nach Schwere der Symptome ein- bis dreimal täglich.

Durch die Behandlung konnte eine deutliche Besserung der Symptome erzielt werden. Es kam zu einer Reduktion des Juckreizes, einer Stabilisierung der Barrierefunktion der Haut und einem Rückgang der geröteten, nässenden bzw. blutenden Ekzeme. In der Regel kam es nach einer Behandlungszeit von vier bis sechs Wochen zur Abheilung des atopischen Ekzems.

Nebenwirkungen wurden nicht beobachtet.

Dieser überraschende und deutliche Behandlungserfolg belegt die Wirksamkeit von Protonenpumpenhemmern bei der Behandlung von dermatologischen Autoim m unerkrankungen.

Des Weiteren wurde die Wirksamkeit der erfindungsgemäßen Zusammensetzung auch bei der Behandlung des Sommerekzems bei Pferden untersucht. Auch hier zeigte sich bei mehreren Tieren eine Abheilung der betroffenen Stellen, auf die die Salbe aufgetragen wurde, und ein Nachwachsen des Fells.

## Patentansprüche

1. Pharmazeutische Zusammensetzung, umfassend einen Protonenpumpenhemmer, zur Verwendung in der Behandlung von dermatologischen Autoimmunerkrankungen, wobei die pharmazeutische Zusammensetzung topisch angewendet wird.

2. Pharmazeutische Zusammensetzung zur Verwendung nach Anspruch 1, wobei der Protonenpumpenhemmer ein substituiertes Pyridylmethylsulfinylbenzimidazol oder dessen pharmazeutisch annehmbares Salz umfasst.

3. Pharmazeutische Zusammensetzung zur Verwendung nach Anspruch 2, wobei der Protonenpumpenhemmer ausgewählt ist aus Omeprazol, Esomeprazol, Lansoprazol, Pantoprazol, deren pharmazeutisch annehmbaren Salzen, und Mischungen hiervon.

4. Pharmazeutische Zusammensetzung zur Verwendung nach einem der vorangehenden Ansprüche, wobei die dermatologische Autoimmunerkrankung atopisches Ekzem oder Psoriasis umfasst.

5. Pharmazeutische Zusammensetzung zur Verwendung nach einem der vorangehenden Ansprüche in Form einer Salbe.

6. Pharmazeutische Zusammensetzung zur Verwendung nach einem der vorangehenden Ansprüche, umfassend 0,1 bis 20 Gew.%, bevorzugt 0,3 bis 10 Gew.%, weiter bevorzugt 1 bis 5 Gew.% des Protonenpumpenhemmers.

7. Pharmazeutische Zusammensetzung zur Verwendung nach einem der vorangehenden Ansprüche, ferner umfassend eine lipophile Trägersubstanz.

8. Pharmazeutische Zusammensetzung zur Verwendung nach einem der vorangehenden Ansprüche, wobei die lipophile Trägersubstanz ausgewählt ist aus synthetischen und mineralischen Wachsen, Fetten und Ölen und deren synthetischen Derivaten, sowie Mischungen hiervon, und/oder aus tierischen und pflanzliche Wachsen, Fetten und Ölen und deren hydrierten oder teilhydrierten Derivaten, sowie Mischungen hiervon.

9. Pharmazeutische Zusammensetzung zur Verwendung nach einem der vorangehenden Ansprüche, wobei die pharmazeutische Zusammensetzung im Wesentlichen wasserfrei ist.

10. Pharmazeutische Zusammensetzung zur Verwendung nach einem der Ansprüche 1 bis 8, wobei die pharmazeutische Zusammensetzung eine w/o-Emulsion oder eine o/w-Emulsion ist.

11. Pharmazeutische Zusammensetzung zur Verwendung nach einem der vorangehenden Ansprüche, ferner umfassend 0,1 bis 5 Gew.% einer alkalischen Substanz und/oder bis zu 5 Gew.% einer oder mehrerer Puffersubstanzen und/oder bis zu 5 Gew.% eines oder mehrerer lipophiler Antioxidantien.

12. Verfahren zur Herstellung einer pharmazeutischen Zusammensetzung, umfassend einen Protonenpumpenhemmer, zur topischen Anwendung, wobei der Protonenpumpenhemmer mit einer lipophilen Trägersubstanz gemischt und diese anschließend mit einem alkalischen Medium behandelt wird.

13. Verfahren nach Anspruch 12, wobei das alkalische Medium eine wässrige alkalische Lösung umfasst.

14. Verfahren nach Anspruch 12 oder 13, wobei die lipophile Trägersubstanz mit dem alkalischen Medium ausgekocht wird.

15. Verfahren nach einem der Ansprüche 12 bis 14, wobei das alkalische Medium nach der Behandlung der lipophilen Trägersubstanz entfernt wird.

## Claims

1. Pharmaceutical composition, comprising a proton pump inhibitor, for use in treating dermatological autoimmune diseases, wherein the pharmaceutical composition is applied topically.

2. Pharmaceutical composition for use according to claim 1, wherein the proton pump inhibitor comprises a substituted pyridylmethylsulfinyl benzimidazole or a pharmaceutically acceptable salt thereof.

3. Pharmaceutical composition for use according to claim 2, wherein the proton pump inhibitor is selected from omeprazole, esomeprazole, lansoprazole, pantoprazole, pharmaceutically acceptable salts thereof, and mixtures thereof.

4. Pharmaceutical composition for use according to any one of the preceding claims, wherein the dermatological autoimmune disease comprises atopic eczema or psoriasis.

5. Pharmaceutical composition for use according to any one of the preceding claims in the form of an ointment.

6. Pharmaceutical composition for use according to any one of the preceding claims, comprising 0.1 to 20 % by weight, preferably 0.3 to 10 % by weight, more preferably 1 to 5 % by weight of the proton pump inhibitor.

7. Pharmaceutical composition for use according to any one of the preceding claims, further comprising a lipophilic carrier substance.

8. Pharmaceutical composition for use according to any one of the preceding claims, wherein the lipophilic carrier substance is selected from synthetic and mineral waxes, fats and oils and their synthetic derivatives, and mixtures thereof, and/or from animal and vegetable waxes, fats and oils and their hydrogenated or partially hydrogenated derivatives, and mixtures thereof.

9. Pharmaceutical composition for use according to any one of the preceding claims, wherein the pharmaceutical composition is substantially anhydrous.

10. Pharmaceutical composition for use according to any one of claims 1 to 8, wherein the pharmaceutical composition is a water-in-oil emulsion or an oil-in-water emulsion.

11. Pharmaceutical composition for use according to any one of the preceding claims, further comprising 0.1 to 5 % by weight of an alkaline substance, and/or up to 5 % by weight of one or more buffering agents, and/or up to 5 % by weight of one or more lipophilic antioxidants.

12. Method for producing a pharmaceutical composition, comprising a proton pump inhibitor, for topical application, wherein the proton pump inhibitor is mixed with a lipophilic carrier substance and this is then treated with an alkaline medium.

13. Method according to claim 12, wherein the alkaline medium comprises an aqueous alkaline solution.

14. Method according to claim 12 or 13, wherein the lipophilic carrier substance is boiled out with the alkaline medium.

15. Method according to any one of claims 12 to 14, wherein the alkaline medium is removed after the treatment of the lipophilic carrier substance.

## Revendications

1. Composition pharmaceutique comprenant un inhibiteur de pompe à protons, pour son utilisation dans le traitement de maladies dermatologiques auto-immunes, la composition pharmaceutique étant appliquée de manière topique.

2. Composition pharmaceutique pour son utilisation selon la revendication 1, dans laquelle l'inhibiteur de pompe à protons contient un pyridylméthylsulfinylbenzimidazole substitué ou ses sels pharmaceutiquements acceptables.

3. Composition pharmaceutique pour son utilisation selon la revendication 2, dans laquelle l'inhibiteur de pompe à protons est choisi parmi l'oméprazole, l'ésoméprazole, le lansoprazole, le pantoprazole, leurs sels pharmaceutiquement acceptables et des mélanges de ces substances.

4. Composition pharmaceutique pour son utilisation selon l'une quelconque des revendications précédentes, dans laquelle la maladie dermatologique auto-immune comprend l'eczéma atopique ou le psoriasis.

5. Composition pharmaceutique pour son utilisation selon l'une quelconque des revendications précédentes, sous forme de pommade.

6. Composition pharmaceutique pour son utilisation selon l'une quelconque des revendications précédentes comprenant de 0,1 à 20 % en poids, de préférence de 0,3 à 10 % en poids, de manière plus préférée de 1 à 5 % en poids d'inhibiteur de pompe à protons.

7. Composition pharmaceutique pour son utilisation selon l'une quelconque des revendications précédentes, comprenant en outre une substance support lipophile.

8. Composition pharmaceutique pour son utilisation selon l'une quelconque des revendications précédentes, dans laquelle la substance support lipophile est choisie parmi des cires, graisses et huiles synthétiques et minérales et leurs dérivés synthétiques, ainsi que des mélanges de ces substances, et/ou des cires, graisses et huiles animales et végétales et leurs dérivés hydrogénés ou partiellement hydrogénés, ainsi que des mélanges de ces substances.

9. Composition pharmaceutique pour son utilisation selon l'une quelconque des revendications précédentes, laquelle est essentiellement anhydre.

10. Composition pharmaceutique pour son utilisation selon l'une quelconque des revendications 1 à 8, laquelle est une émulsion E/H ou une émulsion H/E.

11. Composition pharmaceutique pour son utilisation selon l'une quelconque des revendications précédentes, comprenant en outre de 0,1 à 5 % en poids d'une substance alcaline et/ou jusqu'à 5 % en poids d'une ou plusieurs substances tampons et/ou jusqu'à 5 % en poids d'un ou plusieurs antioxydants lipophiles.

12. Procédé de fabrication d'une composition pharmaceutique comprenant un inhibiteur de pompe à protons pour une application topique, dans laquelle l'inhibiteur de pompe à protons est mélangé à une substance support lipophile et celle-ci est ensuite traitée avec un milieu alcalin.

13. Procédé selon la revendication 12, dans lequel le milieu alcalin comprend une solution aqueuse alcaline.

14. Procédé selon la revendication 12 ou la revendication 13, dans lequel la substance support lipophile est extraite par cuisson avec le milieu alcalin.

15. Procédé selon l'une quelconque des revendications 12 à 14, dans lequel le milieu alcalin est éliminé après le traitement de la substance support lipophile.
